# EUROPEAN PATENT APPLICATION

(11) **EP 2 821 062 A1**
(43) Date of publication of application: **07.01.2015**
(21) Application number: 14174925.9
(22) Date of filing: 30.06.2014
(51) Int. Cl.: A61K 9/00, A61K 9/16, A61K 31/00

(54) **Novel dry powder inhaler formulations**

(30) Priority: 01.07.2013 TR 201307891; 02.09.2013 TR 201310338
(71) Applicant: Arven Ilac Sanayi Ve Ticaret A.S., Istanbul 34460 (TR)
(72) Inventor: Türkyilmaz, Ali, 34460 Istanbul (TR); Mutlu, Onur, 34460 Istanbul (TR); Celik, Devrim, 34460 Istanbul (TR); Orhan, Evrim, 34460 Istanbul (TR); Aygül, Fatih Cengiz, 34460 Istanbul (TR)
(74) Representative: Sevinç, Erkan

(57) **Abstract**

The present invention relates to a formulation for inhalation use to prevent, treat, or alleviate the symptoms of respiratory tract diseases, particularly asthma and chronic obstructive pulmonary disease, as well as to a process for producing this formulation.

## Description

### Field of Invention

The present invention relates to a formulation for inhalation use to prevent, treat, or alleviate the symptoms of respiratory tract diseases, particularly asthma and chronic obstructive pulmonary disease, as well as to a process for producing this formulation.

### Background of Invention

The respiratory tract comprises mainly the lungs and the entirety of the ducts and sacs from the mouth and nose to the alveoli. The respiratory tract has a direct functional relation with the muscle and skeletal system (primarily the diaphragm muscle) providing mechanical ventilation, as well as the cardiovascular system providing blood circulation within the respiratory tract. There are many factors leading to respiratory tract diseases. The factors causing respiratory tract diseases (e.g. chronic obstructive pulmonary disease, lung cancer, cystic fibrosis, asthma, pneumonia, tuberculosis) include genetic factors, age, gender, race, infections, as well as environmental factors such as smoking or exposure to cigarette smoke, air pollution, seasonal factors, geographical conditions, professional factors, etc.

Drugs used in the treatment of respiratory tract diseases are administered through various routes (e.g. inhalation, oral, or parenteral routes). However, the preferred route of administration of these drugs is inhalation, since the drugs are directly delivered to the affected sites (airways) in high doses via this route, have a short onset time, and they lack or have minimal systemic side effects. For this reason, main the inhalation devices, metered dose inhalers, nebulizers and dry powder inhalers have a widespread use.

Among the most frequently encountered problems in the use of metered dose inhalers are the lack of achieving an adequate "hand-inhalation" synchronization in terms of inhaler users, the decline in cognitive functions due to advanced age and mental disorders, and the inability of the users in recognizing the finished state of the inhaler drug due to the lack of a counter. As compared to other inhalation devices, nebulizers are difficult to carry and are expensive. Dry powder inhalers, in turn, are among the most preferred inhalation devices based on some advantages thereof in that they are used conveniently, are environmentally friendly due to the lack of propellants, are carried easily, etc.

The performance of a dry powder inhaler also depends on the formulation delivered to the lungs using this device besides the inhaler characteristics. Formulations which are delivered via dry powder inhalers are in a dry powder form and have an improved stability, an easier use and a more efficient drug delivery capability as compared to those formulations delivered using nebulizers and metered dose inhalers.

The size of the drug particles used in inhalation therapy is closely related to the influence of the respective drug on the respiratory tract disease. In order to gain a therapeutic benefit from the inhaled drug particles, they have to be absorbed in the lungs, i.e. at the bronchial and alveolar sites. For this reason, dry powder formulations prepared for inhalation therapy are formulated using drug particles of a micronized size. The particle size of the drug particles should be below 5 µm for the absorption to take place at these target sites. Since the cohesion forces between the drug particles of this size is extremely high, they tend to agglomerate and thus have weak flow properties. Weak flow properties, in turn, prevent a uniform mixing of the formulation and portioning into blisters, capsules, and reservoirs with a high dose accuracy. This, in turn, causes a high dose variation to occur in the portions such that the amount of dose delivered from an inhaler to a patient differs during each inhalation.

In this respect, it is quite important to improve the flow properties of inhaler formulations in order to guarantee that a correct and consistent amount of active agent is delivered from an inhaler to the lungs of the respective patient any time a dose is inhaled. Having said that, an inhaler formulation having a good flowability further assists the respective inhaler device to function correctly and to provide an uninterrupted performance. In order to overcome the problems described above, the drug particles, i.e. the particles of an active agent, are formulated using carriers for preventing agglomeration and improving the flowability.

However, since the amount of a carrier is quite higher than that of an active agent in dry powder formulations used in inhalation therapy, the properties of the carrier used in the formulation can substantially influence the properties thereof. Therefore, the properties (amount, particle size distribution, etc.) of the carrier used in preparing the dry powder formulation have a great importance in improving the flowability and the uniformity of the formulation.

Various dry powder formulations have been developed according to the prior art for the same or similar objects.

EP0750492B1 discloses a pharmaceutical powder composition comprising micronized drug particles and at least one lactose pellet having a diameter of from 10 to 1500 µm, at least 90% of the lactose particles comprised by the pellet being below 15 µm.

US5478578A discloses a powder for inhalation comprising a micronized active agent and a physiologically acceptable excipient comprising a mixture containing a fine fraction having an average particle size below 10 µm and a coarse fraction having an average particle size between 20 µm and 150 µm, wherein the weight ratio of the micronized active agent to the physiologically acceptable excipient mixture is between 0.01:5 and 0.1:5.

EP0964675B1 discloses a dry powder pharmaceutical composition comprising a mixture of a pharmaceutically active agent and a particulate (roller-dried) anhydrous beta-lactose excipient.

WO9831352A1 discloses a dry powder composition comprising one or more pharmaceutically active substances and a carrier substance, wherein these substances are in a finely divided form and the poured bulk density of the formulation is between 0.28 g/ml and 0.38 g/ml. The same document states that a spheronizing process is performed to bring the poured bulk density of the dry powder composition into the range given above.

Considering the state of the art, it would be quite advantageous to provide a novel dry powder formulation, overcoming the problems described above and meeting all requirements for an efficient treatment of respiratory tract diseases, as well as having a good flowability and uniformity.

### Description of Invention

The present invention relates to a dry powder formulation suitable for use in dry powder inhalers eliminating all problems described above and brining additional advantages to the relevant prior art.

The main object of the present invention is to provide a dry powder formulation with an improved flowability and uniform distribution of the ingredients for use in the prevention, treatment, or in the alleviation of the symptoms of respiretory tract diseases, particularly asthma and chronic obstructive pulmonary disease.

Another object of the present invention is to provide a dry powder formulation portioned at a high dose accuracy and guaranteeing that the amount of an active agent delivered to a patient during the inhalation of each dose is kept under control.

Another object of the present invention is to provide a dry powder formulation in which the active agent delivered to the lungs during inhalation shows a good performance in terms of the fine particle fraction.

Another object of the present invention is to provide a dry powder formulation in which the active agent is provided in each dose in a correct and consistent amount as a result of the content uniformity of the doses portioned into blisters, capsules, or reservoirs.

Another object of the present invention is to provide a method for preparing a dry powder formulation having the features which meet the objects stated above.

A novel dry powder formulation suitable for use in a dry powder inhaler has been developed to carry out all objects referred to above and to be disclosed in the following detailed description.

### Detailed Description of Invention

The present invention relates to a dry powder formulation with an improved flowability and uniform distribution of the ingredients for use in the prevention, treatment, or in the alleviation of the symptoms of respiratory tract diseases, particularly asthma and chronic obstructive pulmonary disease.

In order for an active agent to be carried by a carrier agent in a dry powder formulation for use in inhalation therapy, a sufficiently strong interaction should be present between the particles of the active agent and the particles of the carrier agent. This interaction, however, should both be strong enough to prevent the separation of the particles of the active agent from the particles of the carrier agent during production, transport and storage, and weak enough to release the particles of the active agent from the particles of the carrier agent to be delivered to the lungs upon inhalation. The shape and surface roughness of the particles of the carrier agent have an important influence in meeting this condition. The particles of a carrier agent having a smooth surface will be separated from the particles of the active agent more easily as compared to the particles of a carrier agent of the same size, but having a porous surface structure.

Fine carrier particles are used for promoting the delivery of active agents to the lungs more safely in higher doses. In this context, since the surface energy is not evenly distributed over the carrier particle under normal conditions, the active agent tends to localize on the sites having a higher surface energy. This, in turn, makes it difficult for the active agent to be released from the carrier agent particularly in low-dose formulations. However, when the coarse carrier particles are mixed with the fine carrier particles, the fine carrier particles bind to the higher energy sites on the surface of the coarse carrier particles and cause the surface of the coarse carrier particles to smoothen and gain an even shape such that the active agent binds weakly to these sites.

Accordingly, the carrier agent may be composed of two, three, four or even more particle-size fractions in the dry powder formulation according to the present invention, but preferably two carrier agent fractions are used here, i.e. one is fine particle fraction and other one is coarse particle fraction having different mean particle sizes.

It was surprisingly found that the flowability of the formulation according to the present invention, which comprises a micronized active agent and a carrier agent comprising fine carrier particles and coarse carrier particles, is improved and the active agent is uniformly mixed with the carrier agent, when the weight ratio (M) of the fine carrier particles to the coarse carrier particles is greater than the weight ratio (N) of the active agent particles to the coarse carrier particles in the dry powder formulation. Thus, it is ensured to fill the dry powder formulation into blisters, capsules and reservoirs at a high dose accuracy and to keep under control the amount of active agent delivered from an inhaler to a patient during the inhalation of each dose.

Accordingly, improving the flowability of the dry powder formulation according to the present invention and providing a uniform mixture make it possible to ensure content uniformity in the doses portioned into blisters, capsules and reservoirs, and to deliver a correct and consistent amount of active agent from an inhaler to the patient any time a dose is inhaled.

On the other hand, determining the amount and the particle size of the fine carrier particles, which have an important role in the interaction between the coarse carrier particles and the active agent, in the dry powder formulation is also a critical factor in terms of improving the properties of the formulation. Accordingly, it was observed in the dry powder formulation according to the present invention that the flowability and the uniformity of the formulation reached an optimum level when the weight of the fine carrier particles is below 25% of the total weight of the formulation. The weight of the fine carrier particles in the dry powder formulation according to the present invention is preferably between 0.5% and 15%, more preferably between 1% and 8% of the total weight of the formulation.

According to the present invention, the d_{0.5} particle size by volume defines the maximum particle size of less than 50% by volume of the particles.

According to the present invention, an "emitted dose" refers to the portion of a total dose delivered from an inhaler device to a patient during inhalation. The term "fine particle dose (FPD)", in turn, refers to the amount of dose at an inhalable size (with a particle size below 5 µm) delivered to the lungs. The term "fine particle fraction (FPF)" refers to the percentage of the fine particle dose amount in the total dose. In order to achieve a good performance when dry powder formulations are developed, it is generally intended that the emitted dose is above 85% and the FPF is above 15%.

It was additionally observed that adjusting the ratio of the d_{0.5} particle size by volume of the fine carrier particles to the d_{0.5} particle size by volume of the active agent particles in the dry powder formulation according to the present invention between 25:1 and 1:10, preferably between 20:1 and 1:5, more preferably between 15:1 and 1:1 contributed to bring the flowability and the uniformity of the formulation, as well as the interaction between the coarse carrier particles and the active agent particles to optimal levels, and to deliver a correct and consistent amount of active agent to a patient during each inhalation of a dose and to increase the fine particle fraction (FPF) of the active agent delivered to the lungs of the patient.

Accordingly, the d_{0.5} particle size by volume of the fine carrier particles in the dry powder formulation is between 0.5 µm and 25 µm, preferably between 1 µm and 20 µm, whereas the d_{0.5} particle size by volume of the coarse carrier particles in the dry powder formulation is between 40 µm and 200 µm, preferably between 50 µm and 120 µm.

According to the present invention, the d_{0.5} particle size by volume of the active agent in the formulation is below 5 µm, and particles of this size can provide therapeutic benefit as a result of becoming absorbed at the bronchial and alveolar sites in the lungs.

On the other hand, the particle size of the active agent and the carrier agent used in the dry powder formulation according to the present invention is preferably measured by means of a laser diffraction method. More specifically, the particle size of the active agent is measured according to the present invention using a liquid dispersion method using water as a dispensing agent on a Malvern Mastersizer 2000 Particle Size Analyzer. The particle size of the carrier agent, in turn, is measured using a dry dispersion method using air as a dispensing agent on a Malvern Mastersizer 2000 Particle Size Analyzer. The d_{0.5} particle size of the active agent and of the fine carrier particles and coarse carrier particles in the dry powder formulation are obtained using the measurements described above.

The fine carrier particles and the coarse carrier particles in the dry powder formulation according to the present invention are preferably from the same kind. The carrier agent is selected from a group comprising lactose, mannitol, glucose, trehalose, cellobiose, raffinose, melezitose, lactitol, sucrose, sorbitol, starch, maltitol, or the mixtures thereof; the carrier agent is preferably lactose, more preferably lactose monohydrate.

The active agent in the dry powder formulation according to the present invention is selected from a group comprising salmeterol, formoterol, arformoterol, indacaterol, olodaterol, vilanterol, carmoterol, bambuterol, salbutamol, levosalbutamol, terbutaline, pirbuterol, procaterol, fenoterol, bitolterol, ritodrine, metaproterenol, fluticasone, ciclesonide, budesonide, mometasone, beclomethasone, triamcinolone, flunisolide, dexamethasone, tiotropium, glycopyrronium, ipratropium, aclidinium, oxitropium, or a pharmaceutically acceptable salt or ester thereof, or an enantiomerically pure form thereof, or a racemic mixture thereof, or a combination of two or more thereof; the active agent is preferably selected from formoterol fumarate dihydrate, budesonide or a combination thereof.

The dry powder formulation according to the present invention preferably further comprises at least one additive selected from a group comprising magnesium stearate, stearic acid, sodium lauryl sulfate, sodium stearyl fumarate, stearyl alcohol, sodium benzoate, silicon dioxide (Aerosil), amino acids (leucine, lysine, valine, methionine, or phenylalanine), or the mixtures thereof.

The dry powder formulation is formulated to comprise 1 µg to 4000 µg, preferably 2 µg to 2500 µg, more preferably 3 µg to 1000 µg of the micronized active agent per dose; as well as 25 µg to 50 mg, preferably 50 µg to 40 mg, and more preferably 100 µg to 30 mg of the carrier agent per dose.

In order to achieve another object of the present invention, the dry powder formulation is produced using methods which are known to those skilled person in the art. In the preparation of the dry powder formulation, the active agent particles and the fine carrier particles are provided in a finely divided form within the particle size ranges given above. The components of the formulation can be produced at the corresponding particle sizes given above using methods such as grinding, microparticulation, and direct precipitation which are known to those skilled in the art.

In order to prepare the dry powder formulation, the active agent, the fine carrier particles and the coarse carrier particles are mixed together at the same time or they are added sequentially in any order as the mixing is being continued or first the fine carrier particles and the coarse carrier particles are mixed and then the resulting mixture is mixed with the active agent.

A method for producing the formulation according to the present invention is preferably comprising the following steps:
a. weighing the micronized active agent, the fine carrier fraction and the coarse carrier fraction,
b. mixing the fine carrier fraction and the coarse carrier fraction together for some time period (mixture A),
c. mixing the active agent with mixture A and passing the resulting mixture (mixture B) through a sieve,
d. after mixing the mixture for some more time and then filling the final mixture into suitable packages (blister cavities, capsules or reservoirs).

In order to contribute to a uniform mixing of the powder particles, the active agent and the excipients in the formulation according to the present invention are preferably all sieved and subjected to another mixing process again after they are mixed for the first time. Thus, the resulting mixture is homogenized without causing any change in the particle size thereof. Preferably, an "electrostatic eliminator" is used during the process for reducing the electrostatic charges of the micronized active agent particles.

The dry powder formulation according to the present invention is used in the treatment of respiratory tract diseases selected from asthma and chronic obstructive pulmonary diseases and other obstructive airways diseases. The formulations according to the present invention are particularly used in the treatment, prevention, or in the alleviation of the symptoms of respiratory diseases comprising asthma, acute respiratory distress syndrome, chronic pulmonary inflammatory disease, bronchitis, chronic bronchitis, chronic obstructive pulmonary disease, silicosis, as well as of immune disorders and conditions comprising allergic rhinitis and chronic sinusitis.

The dry powder formulation according to the present invention may be filled into capsules, blisters and reservoirs and administered using any known type of dry powder delivery systems (particularly inhalers). The drug delivery system used may be a single-dose, a multiple-dose or a breath-operated device. Additionally, the present invention further comprises an inhalation kit including a plurality of doses of the dry powder formulation according to the present invention together with a device (e.g. an inhaler) such as a single-dose device, a multiple-dose device or a breath-operated device, wherein the dry powder formulation comprised in the kit is provided in capsules, blisters, or reservoirs.

The present invention is explained in more details in the following examples. These examples are not limiting the scope of the present invention and are to be considered under the light of the foregoing detailed description.

### Examples

### Example 1:

| **Content of Formulation** | **Amount (mg)** | **Amount (%)** | **M** | **N** | **d_{0.5}(1) : d_{0.5}(2)** |
|---|---|---|---|---|---|
| Formoterol fumarate dihydrate (%2 excess dose) | 0.0122 | 0.0490 | 0.0526 | 0.0005 | 2.2:1 |
| Fine lactose monohydrate | 1.2494 | 4.9976 | | | |
| Coarse lactose monohydrate | 23.7384 | 94.9535 | | | |
| Total | 25.0000 | 100.0000 | | | |

In the formulation given above, d_{0.5}(1) defines the maximum particle size of 50% by volume of the fine carrier particles, whereas d_{0.5}(2) defines the maximum particle size of 50% by volume of the active agent particles. Each dose of the formulation given above was filled into a separate capsule and these capsules were analyzed. Accordingly, the active agent content of 10 capsules were analyzed for determining the content uniformity of the capsules comprising the formulation given in the table. According to the results of this analysis, it was determined that the dose comprising the minimum amount of active agent and the dose comprising the maximum amount of active agent contained 95.0% and 104.4%, respectively, of the theoretical active agent amount (without excess) presented in one dose, wherein the mean of all results was 99.8% with a relative standard deviation below 2.

Then, 10 capsules were analyzed using a dose uniformity sampling apparatus (DUSA) to determine the emitted dose. The apparatus was set up to give a pressure drop of 4 kPa and a flow rate and period which would be adequate to provide 4 liters of air. Here, the active agent content of each dose delivered from the inhaler to a patient was determined. Each dose of the formulation given above was filled into a separate capsule and the formulation was inhaled from the capsules in the inhaler during the analysis. Thus, it was found that the lowest amount of active agent and the highest amount of active agent delivered from the inhaler to the patient were 92.8% and 104.2%, respectively, of the theoretical active agent amount (without excess) presented in one dose, wherein the mean of all results was 98.2% with a relative standard deviation below 2.

Additionally, the FPF values were calculated by means of a Next Generation Impactor (NGI) for determining the amount of active agent having a particle size below 5 µm delivered to the lungs of a patient. The device was similarly set up to give a pressure drop of 4 kPa and a flow rate and period which would be adequate to provide 4 liters of air. Each dose of the formulation given above was filled into a separate capsule and the formulation was inhaled from the capsules in the inhaler during the analysis. According to the results of this analysis which was successively performed on 10 separate doses, the FPF was found to be as 38.6% with a relative standard deviation below 2.

### Example 2:

| **Content of Formulation** | **Amount (mg)** | **Amount (%)** | **M** | **N** | **d_{0.5} (1) : d_{0.5} (2)** |
|---|---|---|---|---|---|
| Budesonide (10% excess dose) | 0.2200 | 0.880 | 0.0309 | 0.0092 | 9.5:1 |
| Fine lactose monohydrate | 0.7434 | 2.9736 | | | |
| Coarse lactose monohydrate | 24.0366 | 96.1464 | | | |
| **Total** | 25.0000 | 100.0000 | | | |

In the formulation given above, d_{0.5} (1) defines the maximum particle size of 50% by volume of the fine carrier particles and d_{0.5} (2) defines the maximum particle size of 50% by volume of the active agent particles. Each dose of the formulation given above was filled into a separate capsule and these capsules were analyzed. Accordingly, the active agent content of 10 capsules were analyzed for determining the content uniformity of the capsules comprising the formulation given in the table. According to the results of this analysis, it was determined that the dose comprising the minimum amount of active agent and the dose comprising the maximum amount of active agent contained 97.2% and 103.8%, respectively, of the theoretical active agent amount (without excess) presented in one dose, wherein the mean of all results was 98.5% with a relative standard deviation below 2.

Then, 10 capsules were analyzed using a dose uniformity sampling apparatus (DUSA) to determine the emitted dose. The apparatus was set up to give a pressure drop of 4 kPA and a flow rate and period which would be adequate to provide 4 liters of air. Here, the active agent content of each dose delivered from an inhaler to a patient was determined. Each dose of the formulation given above was filled into a separate capsule and the formulation was inhaled from the capsules in the inhaler during the analysis. Thus, it was found that the lowest amount of active agent and the highest amount of active agent delivered from the inhaler to the patient were 95.8% and 102.8%, respectively, of the theoretical active agent amount (without excess) presented in one dose, wherein the mean of all results was 98.4% with a relative standard deviation below 2.

Additionally, the FPF values were calculated by means of a Next Generation Impactor (NGI) for determining the amount of active agent having a particle size below 5 µm delivered to the lungs of a patient. The device was similarly set up to give a pressure drop of 4 kPa and a flow rate and period which would be adequate to provide 4 liters of air. Each dose of the formulation given above was filled into a separate capsule and the formulation was inhaled from the capsules in the inhaler during the analysis. According to the results of this analysis which was successively performed on 10 separate doses, the FPF was found to be as 25% with a relative standard deviation below 2.

## Claims

1. A dry powder formulation comprising
a. a micronized active agent and
b. a carrier agent comprising fine carrier particles and coarse carrier particles, **characterized in that** the weight ratio M of the fine carrier particles to the coarse carrier particles is greater than the weight ratio N of the active agent particles to the coarse carrier particles.

2. The dry powder formulation according to claim 1, wherein the weight of the fine carrier particles is below 25% of the total weight of the formulation.

3. The dry powder formulation according to claim 2, wherein the weight of the fine carrier particles is between 0.5% and 15% of the total weight of the formulation.

4. The dry powder formulation according to claim 3, wherein the weight of the fine carrier particles is between 1% and 8% of the total weight of the formulation.

5. The dry powder formulation according to any of the preceding claims, wherein the ratio of the d_{0.5} particle size by volume of the fine carrier particles to the d_{0.5} particle size by volume of the active agent particles is between 25:1 and 1:10.

6. The dry powder formulation according to claim 5, wherein the ratio of the d_{0.5} particle size by volume of the fine carrier particles to the d_{0.5} particle size by volume of the active agent particles is between 20:1 and 1:5.

7. The dry powder formulation according to claim 6, wherein the ratio of the d_{0.5} particle size by volume of the fine carrier particles to the d_{0.5} particle size by volume of the active agent particles is between 15:1 and 1:1.

8. The dry powder formulation according to any of the preceding claims, wherein the fine carrier particles and the coarse carrier particles are from the same kind.

9. The dry powder formulation according to claim 8, wherein the fine carrier particles and the coarse carrier particles are selected from a group comprising lactose, mannitol, glucose, trehalose, cellobiose, raffinose, melezitose, lactitol, sucrose, sorbitol, starch, maltitol, or the mixtures thereof.

10. The dry powder formulation according to claim 9, wherein the fine carrier particles and the coarse carrier particles are preferably lactose and more preferably lactose monohydrate.

11. The dry powder formulation according to any of the preceding claims, wherein the active agent is selected from a group comprising salmeterol, formoterol, arformoterol, indacaterol, olodaterol, vilanterol, carmoterol, bambuterol, salbutamol, levosalbutamol, terbutaline, pirbuterol, procaterol, fenoterol, bitolterol, ritodrine, metaproterenol, fluticasone, ciclesonide, budesonide, mometasone, beclomethasone, triamcinolone, flunisolide, dexamethasone, tiotropium, glycopyrronium, ipratropium, aclidinium, oxitropium, or a pharmaceutically acceptable salt or ester thereof, or an enantiomerically pure form thereof, or a racemic mixture thereof, or a combination of two or more thereof.

12. The dry powder formulation according to claim 11, wherein the active agent(s) is preferably formoterol fumarate dihydrate, budesonide, or a combination thereof.

13. The dry powder formulation according to any of the preceding claims, wherein the formulation comprises 1 µg to 4000 µg of the micronized active agent and 25 µg to 50 mg of the carrier agent per dose.
